# FASCICULE DE BREVET EUROPEEN

(11) **EP 1 919 484 B1**
(45) Date de publication et mention de la délivrance du brevet: **19.02.2014**
(21) Numéro de dépôt: 06794360.5
(22) Date de dépôt: 25.08.2006
(51) Int. Cl.: A61K 31/683, A61K 31/685, A61P 11/00, A23L 1/32, A23L 1/29

(54) **UTILISATION DE DHA SOUS FORME DE PHOSPHOLIPIDES POUR LA PREPARATION D'UNE COMPOSITION DESTINEE AU TRAITEMENT DE L'INSUFFISANCE RESPIRATOIRE.**
VERWENDUNG VON DHA IN FORM VON PHOSPHOLIPIDEN ZUR HERSTELLUNG EINER ZUSAMMENSETZUNG ZUR BEHANDLUNG VON RESPIRATORISCHER INSUFFIZIENZ
USE OF DHA IN THE FORM OF PHOSPHOLIPIDS FOR THE PREPARATION OF A COMPOSITION THAT IS INTENDED FOR THE TREATMENT OF RESPIRATORY FAILURE

(30) Priorité: 01.09.2005 FR 0508956
(43) Date de publication de la demande: 14.05.2008
(73) Titulaire: Pieroni, Gérard, 13006 Marseille (FR); Coste, Thierry, 13390 Auriol (FR)
(72) Inventeur: Pieroni, Gérard, 13006 Marseille (FR); Coste, Thierry, 13390 Auriol (FR)
(74) Mandataire: Marek, Pierre
(86) Numéro de dépôt international: PCT/FR2006/001988
(87) Numéro de publication internationale: WO 2007/026071

(56) Documents cités:
- WO-A-02/092540
- FR-A- 2 749 133
- US-B1- 6 482 391
- PATENT ABSTRACTS OF JAPAN vol. 017, no. 342 (C-1076), 29 juin 1993 (1993-06-29) & JP 05 043456 A (SAGAMI CHEM RES CENTER), 23 février 1993 (1993-02-23)
- PATENT ABSTRACTS OF JAPAN vol. 1999, no. 05, 31 mai 1999 (1999-05-31) & JP 11 029470 A (SAGAMI CHEM RES CENTER; HARIMA CHEM INC), 2 février 1999 (1999-02-02)
- PATENT ABSTRACTS OF JAPAN vol. 1996, no. 01, 31 janvier 1996 (1996-01-31) & JP 07 238293 A (BIZEN KASEI KK), 12 septembre 1995 (1995-09-12)
- MICKLEBOROUGH TIMOTHY D ET AL: "Fish oil supplementation reduces severity of exercise-induced bronchoconstriction in elite athletes." AMERICAN JOURNAL OF RESPIRATORY AND CRITICAL CARE MEDICINE. 15 NOV 2003, vol. 168, no. 10, 15 novembre 2003 (2003-11-15), pages 1181-1189, XP002379752 ISSN: 1073-449X cité dans la demande

## Description

Les êtres vivants puisent une partie de leur énergie dans le milieu extérieur. Ils prélèvent dans leur environnement des substances riches en éléments nutritifs ou en énergie, les dégradent (« en extraient » ce qui leur est nécessaire) et y rejettent les reliquats de cette dégradation. La respiration est indispensable à la vie. Les cellules de notre organisme consomment de l'oxygène (O₂) et rejettent du dioxyde de carbone (CO₂), communément appelé gaz carbonique. L'appareil respiratoire est chargé de réaliser les échanges gazeux entre l'organisme (le sang) et l'atmosphère ambiante. Le sang assure le transport des gaz entre l'appareil respiratoire et les cellules.

L'appareil respiratoire se compose de deux éléments principaux :
- une pompe respiratoire (cage thoracique, muscles respiratoires) destinée, tel un soufflet, à faire entrer et sortir l'air des poumons ;
- un diffuseur gazeux (voies aériennes, poumons) qui, au niveau des alvéoles pulmonaires, réalise les échanges O₂ - CO₂ entre le sang et l'air alvéolaire.

L'insuffisance respiratoire se définit comme l'incapacité de l'appareil respiratoire à assumer son rôle, c'est-à-dire assurer une hématose (transformation du sang veineux, chargé en CO₂, en sang artériel, chargé en O₂) normale. Elle peut être chronique (d'installation lente) ou aiguë (d'installation brutale).

L'insuffisance respiratoire peut avoir différentes origines. On reconnaît trois grandes catégories d'insuffisance respiratoire :
- les syndromes obstructifs (bronchite et fumeurs, asthme, mucoviscidose...)
- les syndromes restrictifs (affections neuromusculaires, scolioses, handicaps moteurs, surcharge pondérale...)
- les syndromes mixtes.

A l'heure actuelle le traitement curatif de l'insuffisance respiratoire, en dehors du traitement des syndromes obstructifs par des bronchodilatateurs tels que proposés par HARIMA CHEM INC., Aerosol and médecine for treating pulmonary disease, Patent abstract of Japan du 2 Février 1999 & JP 11029470 A et également HILLS etal., Medicaments for Asthma treatment, US 6,482,391 B1, fait essentiellement appel à une aide mécanique à la respiratoire et/ou à l'apport d'oxygène dans les cas d'encombrement bronchique important.

L'ensemble des solutions proposées fait ainsi appel à des procédés visant à accroître l'apport d'oxygène à l'organisme. Par exemple la demande de brevet japonais JP11 029470 du 2 Février 1999, décrit l'utilisation de phospholipides de DHA, pour une administration sous la forme d'aérosol, pour le traitement de maladies du poumon lors de crises aiguës. Le traitement décrit ne s'inscrit pas dans le long terme et ne vise qu'à améliorer à court terme l'apport d'oxygène à l'organisme.

Aucun des procédés décrits dans l'art antérieur ne vise à améliorer la captation de l'oxygène et son transfert dans l'organisme par le sang, particulièrement sur le long terme.

Il n'existe pas, à l'heure actuelle, de médication spécifique à visée symptomatique pour la prévention de l'insuffisance respiratoire chez l'homme, ayant fait la preuve de son efficacité et de son innocuité. Bien que dès 1991 l'utilisation d'un acide gras n-3 à longue chaine tel que l'acide docosahexaénoïque (DHA) ait été proposée pour inhiber la production de PAF (Platelet Agregating Factor), très vasoconstricteur, dans le cas de l'asthme, et cela sans aucune précision sur la durée du traitement ; SAGAMI CHEM RES CENTER, Agent for inhibiting production of Platelet Activating Factor, Patent Abstracts of Japan du 13 Août 1991 & JP 05043456A. On sait que les acides gras n-3 à longue chaîne (AGPI-LC n-3) tels que l'acide eicosapentaénoïque (EPA ; C20 :5n-3) et l'acide docosahexaénoïque (DHA ; C22 :6n-3) ont des actions multiples sur les membranes cellulaires, en particulier sur la fluidité de la membrane des globules rouges, et qu'ils sont également susceptibles d'agir sur la contractilité vasculaire et le rythme cardiaque. Or, l'approvisionnement en oxygène de l'organisme dépend de l'hémoviscosité, qui est liée à la déformabilité des globules rouges, et de la vasodilatation des vaisseaux qui régulent la micro circulation périphérique.

Les AGPI-LC n-3 sont donc potentiellement des agents pouvant moduler l'oxygénation des tissus et donc la respiration.

C'est ainsi qu'il a été montré qu'un apport journalier de 3g d'AGPI-LC n-3 (1,0g d'EPA et 1,2g de DHA) sous forme d'huile de poisson, soit un apport bien supérieur à la limite d'apport maximal de 2g/j d'AGPI-LC n-3 recommandée par l'AFSSA (Agence Française pour la Sécurité Sanitaire des Aliments), conduisait à une amélioration de la capacité respiratoire maximale (VO₂max) de sportifs uniquement après un effort d'endurance important (80 min à 70% de leur VO₂max). Mais qu'en l'absence d'effort physique de longue durée la prise de 3g/j d'AGPI-LC n-3 n'a aucun effet (Léger CL et coll, Cah.Nutr.Diót, XXVII, 2, 1002). Ccoi a été confirmé par ailleurs ; Miokloborough TD et coll, Am J. Respir. Crit. Care Med. 168(10),1181-9 (2003) ont montré que la prise journalière de 3,2g d'EPA et de 2,2g de DHA sous forme d'huile de poisson n'a aucun effet sur la fonction pulmonaire avant effort chez des athlètes d'élite.

Or les inventeurs ont, maintenant et de manière surprenante, montré qu'une supplémentation per os en DHA seul, sous forme de phospholipides, à une dose inférieure à 200 mg/jour, permet à des insuffisants respiratoires, incapables de part leur état de réaliser le moindre effort physique de longue durée, d'améliorer de façon parfois spectaculaire leur capacité à se mouvoir. Ces améliorations sont liées à la prise de la supplémentation, elles disparaissent peu de temps après l'arrêt de celle-ci, pour réapparaître avec la reprise de la supplémentation.

Chez l'homme, les supplémentations nutritionnelles à base de DHA, sous forme de phospholipides, dans le but de prévenir les effets de l'insuffisance respiratoire n'ont jamais été décrites.

Ainsi, la présente invention concerne l'utilisation d'acide docosahexaénoïque (DHA ; C22:6 n-3) sous forme de phospholipides pour la préparation d'une composition destinée à la prévention de l'insuffisance respiratoire.

Le DHA est un acide gras de la famille des oméga 3 comprenant une chaîne carbonée de 22 atomes de carbone et six doubles liaisons en cis (C22:6 n-3) dont la formule est la suivante :

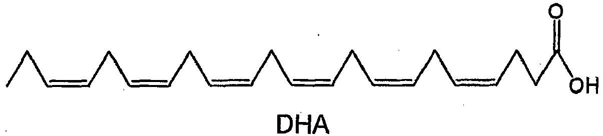

Il convient de préciser que le DHA est un acide gras auquel on attribue depuis longtemps un rôle protecteur vis-à-vis des risques cardiovasculaires et de dépression. Il est également recommandé aux femmes enceintes pour le bon développement du foetus. De manière générale, les nutritionnistes conseillent de consommer environ 2 mg/kg/jour de DHA pour être en bonne santé [Apports nutritionnels conseillés pour la population française, Agence Française pour la Sécurité Sanitaire des Aliments, Editions TEC et DOC, Paris, 2001]. Il existe même des régimes spécifiques, notamment à base de poisson, permettant d'augmenter significativement les apports de DHA dans l'alimentation.

Pour autant, les régimes alimentaires riches en DHA n'ont jamais montré d'effets particuliers sur l'insuffisance respiratoire.

C'est donc de manière très inattendue que les inventeurs ont constaté que certains extraits d'oeufs enrichis en DHA, permettent d'améliorer de façon notable les symptômes de l'insuffisance respiratoire.

En outre, il a été constaté que des doses élevées de DHA sous forme d'huiles de poisson ont tendance à provoquer des lourdeurs d'estomac et des diarrhées, ce qui accentue les risques d'entéropathie [Bums C.P. et al., Phase I clinical study of fish oil fatty acid capsules for patients with cancer cachexia : cancer ànd leukemia group B, study 9473, 1999, Clin. Cancer Res. 5(12) : 3942-3947].

Or les inventeurs ont montré que des doses de DHA aussi faibles que 200 mg/jour de DHA suffisaient à apporter aux insuffisants respiratoires une amélioration de leur condition de vie.

En outre, les inventeurs, en analysant différents paramètres de l'insuffisance respiratoire, ont réalisé que les phospholipides-DHA provenant des oeufs, principalement des lécithines, présentent des propriétés vis-à-vis de l'insuffisance respiratoire que ne présentent pas les autres formes de DHA, en particulier celles sous forme de triacylglycérides dans leur aptitude à modifier la composition en acides gras des membranes des globules rouges.

L'objet de la présente invention consiste donc en l'utilisation d'au moins un phospholipide de formule générale (I) 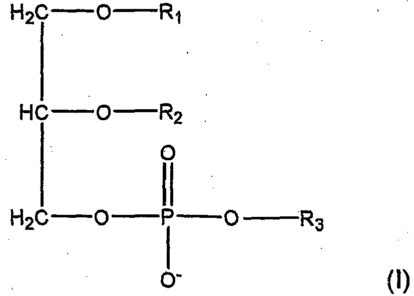 dans laquelle au moins un des groupes R₁ ou R₂ représente un reste docosahexaénoyle (DHA), l'autre représentant, le cas échéant, un reste acyle saturé ou insaturé comprenant de 8 à 24 atomes de carbone, et le groupe R₃ représente un reste choline, sérine, éthanolamine, inositol, glycérol ou hydrogène, pour ta préparation d'une composition entérale destinée au traitement et/ou à la prévention de l'insuffisance respiratoire chez un patient, particutièrement un humain, à une dose de 200 mg/jour de DHA.

Un intérêt de l'invention réside dans le fait que le traitement ou la prévention de l'insuffisance respiratoire ne nécessite l'administration que d'une dose équivalente de DHA comprise entre 0,1 et 2,5 mg/kg/jour, pour un individu de corpulence moyenne d'environ 75 kg.

On entend par "dose équivalente de DHA", la quantité en poids de reste DHA de formule II : 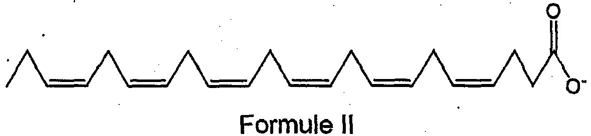 apportée par les phospholipides de formule (I).

On entend par phospholipide de formule (I) un lipide complexe formé de glycérol, dont deux fonctions alcool sont estérifiées par des acides gras (groupes R₁ et R₂), la troisième étant estérifiée par de l'acide phosphorique, lui-même lié à divers composés (groupe R₃).

Préférentiellement selon l'invention, le reste docosahexaénoyle (DHA) est en position R₂.

Sous une forme particulière de l'invention, lorsque l'un des groupes R₁ ou R₂ est un reste acyle, alors il peut être choisi parmi un reste palmitate, stéarate, oléate, linoléate ou arachidonate, préférentiellement un reste palmitate.

Sous une autre forme particulièrement préférée de l'invention, le groupe R₃ représente un reste correspondant à la choline de formule :

-CH₂CH₂N⁺(CH₃)₃.

Les phospholipides utilisables selon l'invention se présentent généralement sous forme de mélanges de différents types de phospholipides de formule (I), et consistent notamment en des lécithines-DHA, c'est-à-dire des complexes de lipides phosphorés combinées à des huiles, où prédominent des glycérophospholipides choliniques de formule (I). Ces lécithines sont amphotères, solubles dans l'alcool, précipitent dans l'acétone et forment une émulsion avec l'eau. De telles lécithines sont présentes plus particulièrement dans les oeufs, lesquels peuvent être enrichis ou non en DHA.

Une méthode pour obtenir des lécithines-DHA, notamment à partir d'oeufs d'oiseau dont l'alimentation est enrichie en DHA, est décrite dans la demande de brevet FR2749133. Les lécithines DHA préparés selon cette méthode sont particulièrement efficaces aux fins de l'utilisation selon l'invention. L'huile de jaune d'oeuf riche en DHA, sous forme de phospholipides telle que décrite par BIZEN KASEI KK, Production of docosahexaenoic acid-containing egg yolk oil, Patent Abstracts of Japan du 01 Mars 1994 & JP 07238293A pourrait ainsi trouver une application originale.

Un aspect préféré de l'invention consiste donc en l'utilisation d'au moins un phospholipide de formule (I) dans laquelle ledit phospholipide est extrait d'oeufs, plus particulièrement d'oeufs enrichis en DHA.

Généralement la dose équivalente de DHA, rapportée en masse de poids corporel et par jour est comprise entre 0,1 et 2,5 mg/kg/jour, et plus généralement entre 0,3 et 2mg/kg/jour. Ces doses correspondent respectivement à des doses moyennes de phospholipides de formule (I) rapportées en masse de poids corporel et par jour, comprises respectivement entre environ 1 et 70 mg/kg/jour, généralement entre environ 3,5 et 50 mg/kg/jour et plus généralement entre environ 7,5 et 25mg/kg/jour (masse brute de phospholipides).

Les phospholipides de formule (I) peuvent être utilisés tels quels, c'est-à-dire substantiellement non associés à d'autres phospholipides ou acides gras. Cependant, du fait qu'il est plus facile d'extraire ces phospholipides de produits naturels, lesdits phospholipides sont, le plus souvent, mis-en-oeuvre sous la forme de complexes comprenant d'autres phospholipides et/ou des acides gras.

Selon l'invention, les phospholipides de formule (I) peuvent être utilisés sous forme d'hydrolysats partiels ou totaux. Ces hydrolysats comprennent, en général, du DHA libre et, le cas échéant, d'autres acides gras issus des phospholipides de formule (I). De tels hydrolysats sont obtenus par des méthodes classiques, par exemple, en faisant agir des enzymes telles que la phospholipase pancréatique et la lipase pancréatique sur les phospholipides de formule (I).

Par composition entérale on entend selon l'invention une composition permettant d'introduire un actif par la voie digestive, à l'exception de toute autre voie, particulièrement la voie respiratoire. Une composition entérale utilisable selon l'invention peut revêtir différentes formes, et peut consister, notamment, en une composition pharmaceutique solide ou liquide sous forme de poudre, de comprimés simples ou dragéifiés, de gélules, de capsules molles, de granulés, de caramels, de suppositoires, ou de sirops.

Compte tenu du fait que les phospholipides de formule (1) décrits plus haut ne présentent pas de toxicité pour l'homme, la composition selon l'invention peut prendre la forme d'un complément alimentaire. Un tel complément alimentaire est particulièrement indiqué pour améliorer les conditions de vie des personnes présentant une insuffisance respiratoire, et cela, même avant que les premiers symptômes de la maladie ne soient constatés.

Sous une autre forme particulière de l'invention, la composition peut être incorporée à un aliment et ainsi enrichir celui-ci en DHA.

L'exemple donné ci-après entend illustrer l'invention de manière non limitative.

### EXEMPLE

On évalue l'effet du traitement «phospholipides» consistant en un apport de 140 mg/jour de DHA sous forme de poudre de jaune d'oeuf (10 g/jour) présents très majoritairement sous la forme de phospholipides de DHA sur des individus sujets à l'insuffisance respiratoire. C'est la tendance à l'essoufflement qui est évaluée, lors d'un passage d'une condition de repos à une condition d'effort.

Par condition d'effort on entend dans le présent test le passage du repos à un effort proportionnel aux capacités reconnues des personnes. L'amélioration s'estime sur la tendance à l'essoufflement de l'individu en condition d'effort.

Le placebo utilisé consiste en des capsules molles d'ester éthylique de DHA (175 mg/jour). Les esters d'acides gras sont légèrement bien moins métabolisés que les formes triacylglycéride et phospholipide d'où l'utilisation d'une dose 25% plus élevée pour le placebo.

Le protocole suivant est mis en oeuvre :
5 personnes, 3 femmes et 2 hommes, d'âges compris entre 60 et 83 ans, de poids compris entre 72 et 109 kg et de tailles comprises entre 145 et 175 cm, ont reçu le placebo pendant 3 semaines. Au terme de cette supplémentation un bilan de leur insuffisance respiratoire est fait.

Puis, les 5 personnes reçoivent alors pendant 3 semaines le traitement «phospholipides» et un nouveau bilan de leur insuffisance respiratoire est réalisé.

Puis, la supplémentation en phospholipide DHA est arrêtée pendant 6 semaines, au bout desquelles un nouveau bilan est réalisé.

Puis la supplémentation est reprise pendant 3 semaines et un bilan est réalisé.

Le tableau ci-dessous résume la sévérité de l'insuffisance respiratoire des personnes avant ou après supplémentation, avec le placebo ou la composition selon l'invention.

| | Dyspnée | | | | | |
|---|---|---|---|---|---|---|
| | Avant traitement | | Après traitement | | | |
| | | | Placebo | | «phospholipides» | |
| Patient | Au repos | A l'effort | Au repos | A l'effort | Au repos | A l'effort |
| A | ++ | ++ | ND | | + | + |
| B | + | ++ | + | ++ | 0 | + |
| C | 0 | + | 0 | + | 0 | 0 |
| D | + | ++ | + | ++ | 0 | 0 |
| E | + | ++ | + | ++ | 0 | 0 |

| | | | | | | |
|---|---|---|---|---|---|---|
| 0 : pas d'insuffisance respiratoire + : insuffisance respiratoire légère ++ : insuffisance respiratoire forte ND : non déterminé | | | | | | |

Ces résultats montrent une diminution, voire une disparition, de la tendance à l'essoufflement des personnes ayant reçu une supplémentation en phospholipides-DHA, que ce soit, après supplémentation en condition de repos ou en condition d'effort.

Ces résultats vont dans le sens d'une supplémentation en phospholipides-DHA pour améliorer les conditions de vie des insuffisants respiratoires.

Il est intéressant de noter que lorsque la supplémentation en phospholipides-DHA est arrêtée, les personnes retrouvent leurs conditions d'avant la supplémentation et lorsque la supplémentation est reprise les améliorations constatées avant l'arrêt sont reproduites. L'effet de la supplémentation en phospholipides-DHA est donc transitoire et ne peut être considéré comme un traitement thérapeutique.

## Revendications

1. Utilisation d'au moins un phospholipide de formule générale (I) 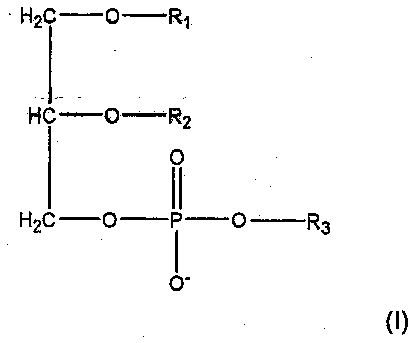 dans laquelle au moins un des groupes R₁ ou R₂ représente un reste docosahexaénoyle (DHA), l'autre représentant, le cas échéant, un reste acyle saturé ou insaturé comprenant de 8 à 24 atomes de carbone, et le groupe R₃ représente un reste choline, sérine, éthanolamine, inositol, glycérol ou hydrogène, sous forme d'oeuf enrichi en DHA ou d'extraits d'oeuf enrichi en DHA, pour la préparation d'une composition entérale destinée au traitement et/ou à la prévention de l'insuffisance respiratoire chez un patient, particulièrement un humain, à une dose de 200 mg/jour de DHA.

2. Utilisation d'au moins un phospholipide de formule (I) selon la revendication 1, dans laquelle le groupe R₂ représente le reste docosahexaénoyle (DHA).

3. Utilisation d'au moins un phospholipide de formule (I) selon l'une quelconque des revendications 1 ou 2, dans laquelle lorsque l'un des groupes R₁ ou R₂ est un reste acyle, alors il peut être choisi parmi un reste palmitate, stéarate,oléate, linoléate ou arachidonate.

4. Utilisation d'au moins un phospholipide de formule (I) selon l'une quelconque des revendications 1 à 3, dans laquelle le groupe R₃ représente un reste choline de formule : -CH₂CH₂N⁺(CH₃)₃.

5. Utilisation d'au moins un phospholipide de formule (I) selon l'une quelconque des revendications 1 à 4, dans laquelle les phospholipides de formule (I) consistent en des lécithines-DHA.

6. Utilisation selon l'une quelconque des revendications 1 à 5, dans laquelle le phospholipide de formule (I) est présent sous la forme d'un hydrolysat total ou partiel.

7. Utilisation selon l'une quelconque des revendications 1 à 6, dans laquelle ladite composition est une composition pharmaceutique solide ou liquide, sous forme de poudre, de comprimés simples ou dragéifiés, de gélules, de capsules molles, de granulés, de caramels, de suppositoires, ou de sirops.

8. Utilisation selon l'une quelconque des revendications 1 à 7, dans laquelle ladite composition est un complément alimentaire.

## Patentansprüche

1. Verwendung von mindestens einem Phospholipid der allgemeinen Formel (I) 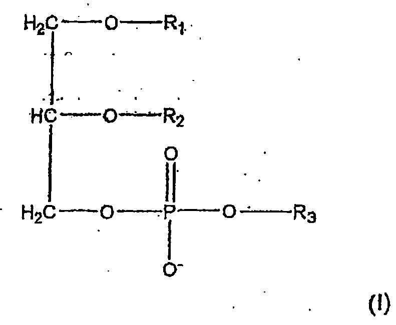 worin mindestens eine der Gruppen RI oder R2 einen Docosahexaensäure-Rest (DHA) darstellen, wobei der andere Vertreter, gegebenenfalls, einen gesättigten oder ungesättigten Acylrest mit 8 bis 24 Kohlenstoffatomen, und die Gruppe R3 ein Rest Cholin, Serin, Ethanolamin, Inositol, Glycerin oder Wasserstoff darstellen, in der Form von DHA-angereicherten Eiern oder mit DHA-angereicherten Ei-Extrakten, zur Herstellung einer Zusammensetzung, für die Behandlung und / oder Prävention von Lungenversagen bei einem Patienten, insbesondere einem Menschen in einer Dosis von 200 mg/Tag von DHA.

2. Verwendung von mindestens einem Phospholipid der Formel (I) nach Anspruch 1, worin die Gruppe R2 den Docosahexaensäure-Rest (DHA) darstellt.

3. Verwendung von mindestens einem Phospholipid der Formel (I) nach einem der Ansprüche 1 oder 2, wobei, wenn einer der Reste der Gruppen RI und R2 einen Acylrest darstellen, so kann ein Rest aus Palmitat, Stearat, Oleat, Linoleat oder Arachidonat ausgewählt werden.

4. Verwendung von mindestens einem Phospholipid der Formel (I) nach einem der Ansprüche 1 bis 3, wobei die Gruppe R3 eine Cholin-Rest der Formel: - CH₂CH₂N⁺(CH₃)₃ darstellt.

5. Verwendung von mindestens einem Phospholipid der Formel (I) nach einem der Ansprüche 1 bis 4, wobei das Phospholipid der Formel (I) aus DHA-Lecithinen besteht.

6. Verwendung nach einem der Ansprüche 1 bis 5, worin das Phospholipid der Formel (I) in der Form eines vollständigen oder partiellen Hydrolysats vorliegt.

7. Verwendung nach einem der Ansprüche 1 bis 6, wobei die Zusammensetzung eine feste oder flüssige pharmazeutischen Zusammensetzung in Pulverform, in Form von einfachen oder beschichteten Tabletten, Kapseln, weichen Kapseln, Granulaten, Karamellen, Zäpfchen, Sirups oder Injektionen ist.

8. Verwendung nach einem der Ansprüche 1 bis 7, wobei die Zusammensetzung ein Nahrungsergänzungsmittel ist.

## Claims

1. Use of at least one phospholipid of the general formula (1) 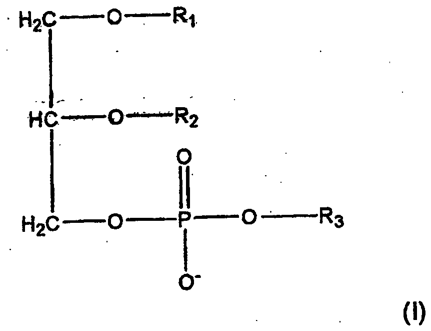 in which at least one of the groups R₁ or R₂ represents a docosahexaenoyl (DHA) residue and the other represents, as appropriate, a saturated or unsaturated acyl residue containing 8 to 24 carbon atoms, and group R₃ represents a choline residue, a serine residue, an ethanolamine residue, an inositol residue, a glycerol residue,or hydrogen, in the form of DHA-enriched egg or extracts of DHA-enriched egg, for the preparation of an enteral composition intended to treat and/or prevent respiratory insufficiency in a patient, especially human, at a dose of 200 mg/day of DHA.

2. Use of at least one phospholipid of formula (I) according to claim 1, in which groupR₂ represents the docosahexaenoyl (DHA)residue.

3. Use ofat least one phospholipid of formula (I) according to any of claims 1 or 2, in which when one of the groups R₁ or R₂ is an acyl residue, it may be selected from a palmitate residue, a stearate residue, anoleate residue, a linoleate residue, or an arachidonate residue.

4. Use of at least one phospholipid of formula (I)according to any of claims 1 to 3, in which the group R₃ represents a choline residue of formula:
-CH₂CH₂N⁺(CH₃)₃.

5. Use of at least one phospholipid of formula (I)according to any of claims 1 to 4, in which the phospholipids of formula (I) consist of DHA lecithins.

6. Use according to any of claims 1 to 5 in which the phospholipid of formula (I) is present in partially or fullyhydrolysed form.

7. Use according to any of claims 1 to 6, in which said composition is a solid or liquid pharmaceutical composition in the form of powder, plain or sugar-coated tablets, gelules, soft capsules, granules, lozenges, suppositories, or syrups.

8. Use according to any of claims 1 to 7, in which said composition is a food supplement.
